# EUROPEAN PATENT APPLICATION

(11) **EP 4 434 484 A1**
(43) Date of publication of application: **25.09.2024**
(21) Application number: 23164201.8
(22) Date of filing: 24.03.2023
(51) Int. Cl.: A61B 34/20, A61B 90/50

(54) **SYSTEM FOR CONTROLLED PLACEMENT OF AN OBJECT IN SPACE USING A SPATIAL ALIGNMENT DEVICE**

(71) Applicant: CAScination AG, 3008 Bern (CH)
(72) Inventor: Matulic, Marco, 3008 Bern (CH)
(74) Representative: Schulz Junghans Patentanwälte PartGmbB

(57) **Abstract**

A spatial alignment system comprising a spatial alignment tool, a computer and software containing an alignment algorithm is provided. The spatial alignment system provides a series of steps for placement of an object to a user on a user interface. The provided series of steps allow the user to move the alignment tool in defined increments through multiple degrees of freedom to accomplish placement of the object in a desired position and orientation. The system provides for precise, predictable and controllable movement of the alignment tool to allow for correct placement of the object. The spatial alignment system may be configured for movement of the spatial alignment tool to facilitate accurate and predicable placement of a surgical tool and/or a medical device. In some embodiments, the system allows for controlled and predictable placement of medical devices such as cochlear implants. The spatial alignment tool may incorporate means for a user to cause movement through defined increments wherein the means provide haptic feedback to the user to confirm the desired movements. Provided is thus an inventive system that helps ensure quality placement of medical devices such as cochlear implants.

## Description

### FIELD OF THE INVENTION

The invention relates to a system comprising a spatial alignment device incorporating a feedback system. In certain embodiments, the spatial alignment device with a feedback system may allow for physical alignment along more than one and as many as seven or eight degrees of freedom. In particular, each axis of motion may be comprised of a self-locking gear, an input joint, an output joint and an element for controlling the axis of motion that provides haptic feedback to a user. In various embodiments, the inventive system may include a user interface that displays a series of steps for moving an object to be placed by the spatial alignment device along the multiple degrees of freedom to a desired position and orientation. The inventive system may also include in certain embodiments software comprising an algorithm with a kinematic model of the spatial alignment device that can provide a user with a series of steps for moving an object to a desired position and orientation.

In particular, the present invention pertains to a system including a spatial alignment device for precise and controllable movement of an object along multiple degrees of freedom so that the object reaches and maintains a desired position and orientation.

In certain embodiments, the object may be a tool such as a surgical tool that can be used to conduct a certain surgical task or an active surgical tool that acts a connector to a medical implant, wherein the medical implant must be moved predictably and controllably through the active surgical tool and to a precisely arranged position and orientation achieved by the spatial positioning stage.

More particularly, the instant invention may relate to the movement of surgical tools and/or medical implants to particular positions and orientations by a system that provides a set of steps to a user on graphical user interface, and control means for the user to carry out the provided steps by causing motion along the multiple degrees of freedom. Most particularly, the inventive system comprises a spatial alignment device, a computer and software wherein the software provides a set of instructions to a user for manipulating the spatial alignment device to cause placement of a surgical tool and/or medical implant to a specific position and orientation.

### BACKGROUND OF THE INVENTION

In many medical and non-medical applications, placement of a device or object in a specific position and orientation is required. This can be particularly true in medical applications, and especially in surgical applications, where placement of a surgical tool or medical implant is needed in a specific position and orientation to facilitate medical or surgical treatment.

In the current state of the art, alignment systems most commonly present a user with a crosshair and the user can adjust the position of an object along one or more degrees of freedom by means of robotic manipulators, articulated manually operatable arms or the like. This adjustment process may or may not be controlled/supervised by a tracking system. The alignment system typically provides a cross-hair viewer that assists the user in general. However, in currently known systems, the user may not be told by the alignment system which specific degrees of freedom need to be adjusted at a given point in time, and by how much translation (e.g., in mm) or orientation (e.g., in degrees), to place an object in a desired position and orientation.

For example, current alignment systems may simply present a user with a crosshair-type targeting apparatus where the user attempts to move the crosshair to the desired position and orientation. Hence, any cross-hair viewer (or similar) only provides information about the current state of displacement, but does not construct /calculate any type of user oriented recommendations for future actions towards solving the spatial alignment problem.

This type of system lacks guidance in the form of any particular set of instructions, lacks any sort of haptic feedback and, accordingly, lacks accuracy. Thus, suitability of a system like this for precise medical applications is very much in doubt.

More particularly, state of the art systems may also not provide the user with means to control the speed, precision and predictability of movement of an object through the various degrees of freedom. Movement in any particular degree of freedom may not be broken down by the system into increments or particular steps or particular distances. Accordingly, movement of an object with current spatial alignment systems may not be predictable or precise.

Currently available systems thus may not present a user with a set of specific uer oriented instructions (on a graphical user interface or otherwise) for moving the output side of an alignment system to a desired target (position and orientation) and may not tell the user how far and how fast to move the alignment system across multiple degrees of freedom. Current systems are also not known to provide users with the means to control spatial positioning that include haptic feedback, so that the user knows when it has moved an object to be placed through one or more defined increments in one or more degrees of freedom.

There are many applications, including medical applications, where it would be useful to provide a system that would give a user a set of instructions for moving an object in defined increments along multiple degrees of freedom to desired position and orientation, while allowing for haptic feedback to the user so it knows when it has caused an alignment system to move in the defined increments. In particular, a user may with to place a spatial alignment device held by a surgical arm in a particular position and orientation for further placement of a surgical tool and/or medical implant with respect to a patient.

Keeping in mind the identified drawbacks of known spatial alignment systems, the current inventors provide a spatial alignment system that provides its users with a set of steps for manipulating an alignment device through defined increments and degrees of freedom so as to place an object in a desired position and orientation. The inventive system can be used for the placement of surgical tools and medical implants. In particular the inventive system can be used to assist in the placement of cochlear implants, solely by way of example in an embodiment wherein a surgical arm holds a spatial alignment device which in turn can hold a surgical tool connected to a cochlear implant, and wherein the spatial alignment device can be manipulated in a controlled, predictable manner to align the electrode of a cochlear implant in a desired position and orientation and upon which the surgical tool can then carry out a specific motion pattern along the previously obtained trajectory with the intent to advance the cochlea implant electrode slowly and steadily into the cochlea.

Those of skill in the art will realise of course that the same need is felt in any surgical field requiring precise movement control and/or precise placement of medical implants. More broadly, scientific or manufacturing fields requiring precise movement control but also precise placement of components will also benefit.

### SUMMARY OF THE INVENTION

These aims and other advantages are achieved by a new spatial alignment system comprising a spatial alignment device with a feedback system for precise and controllable movement of an object along multiple degrees of freedom. The spatial alignment device of the current system is optimally configured to control movement of an object along at least five degrees of freedom. In particular embodiments, the at least five degrees of freedom comprise at least three translational degrees of freedom and at least two rotational degrees of freedom. One of skill in the art will understand that the at least five degrees of freedom may be at least seven or eight degrees of freedom, or more, while staying within the spirit of the current invention. The skilled artisan will know how many degrees of freedom of controlled movement are required for their particular application, whether medical or non-medical.

In various embodiments of the current system, the spatial alignment device is configured to control movement of an object along the at least five degrees of freedom so that the object reaches a desired position and orientation. Solely by way of example, in a medical application, the spatial alignment device may be configured to move a surgical tool connected to a medical implant to a desired position and orientation with respect to an intervention site on a human patient.

In various embodiments, the feedback system of the spatial alignment device may incorporate means for a user to understand that they have caused movement of an object along one or more degrees of freedom with the spatial alignment device through one or more defined increments. Solely by way of example, the spatial alignment device may be equipped with a number of input means such as knobs or buttons matching the number of degrees of freedom in which it can provide controlled motion of an object. The user may thus be able to manipulate the knobs or buttons to cause movement of the object along one or more desired degrees of freedom through one or more defined increments. Movement through each increment through manipulation of the relevant knob or button by the user may cause, for example, an audible click or a beep or a form of haptic feedback that informs the user that they have moved the object through a defined increment along a particular degree of freedom through the operation of the spatial alignment device.

The spatial alignment system incorporating the inventive spatial alignment device may further comprise a computer containing software for operation of the spatial alignment device. In some embodiments, the computer and software may provide a set of instructions to the user for manipulating the spatial alignment device to provide controlled movement of an object along multiple degrees of freedom to achieve a desired placement of the object. This desired placement of the object may include placing the object in a desired position and/or orientation. More particularly, the set of instructions to the user may include instructions to manipulate input means of the spatial alignment device to control motion through a number of defined increments along multiple degrees of freedom to achieve a desired position and orientation of an object.

In various embodiments, the inventive system incorporating the spatial alignment device, computer and software may further include a graphical user interface upon which instructions to a user for manipulation of the spatial alignment device are displayed. For example, the inventive system may display a set of instructions to a user for manipulating the input means of the spatial alignment device to cause movement of an object along multiple degrees of freedom and through multiple defined increments so as to place the object at a desired position and orientation. Optionally, the instructions may be presented on the graphical user interface in a step-wise or sequential fashion. Solely by way of example, the user may be instructed to manipulate the appropriate input means to move the object in five defined increments along an x-axis, followed by 7 defined increments along a y-axis, and then followed by 6 defined increments along a rotational axis.

In various embodiments, the instructions may be presented to the user on a graphical user interface as a set of text instructions. In other embodiments, the instructions may be presented to the user in pictorial means, for example with a graphical representation of the input means to be manipulated along with an arrow representing the direction of manipulation and a number representing the number of defined increments to be moved. In this way, with the inventive system including feedback means, the user is able to follow the instructions and know through haptic or other feedback that they have carried out the instructions.

In various embodiments, the inventive system incorporating the spatial alignment device, computer, software and graphical user interface also includes a kinematic model of the spatial alignment device. Accordingly, if the inventive system is provided with a starting position and orientation in cartesian space of an object held by the spatial alignment device and a desired position and orientation in cartesian space of the object, it is able to compute, using the kinematic model, the number of increments in each degree of freedom that must be moved in order to place the object in its desired position and orientation.

In various embodiments of the present inventive system, the object held by the spatial alignment device may be a tool useful for a particular application. In some applications, the tool may be a surgical tool useful carrying out a particular surgical procedure.

In particular embodiments, the tool held by the spatial alignment device may be an insertion device that moves a medical implant forward or backward along a particular axis. Solely by way of example, the tool held by the spatial alignment device may be an insertion device that holds a cochlear implant through means of a forceps, whereby the insertion device is configured to advance or retract the cochlear implant in the inner ear of a patient. In these particular embodiments, the current inventive system may be configured to allow placement of the insertion device in a desired position and orientation to allow for optimal advancement of a cochlear implant into the cochlea of a patient. Accordingly, the user of the inventive system would be provided with a set of instructions for manipulating one or more input means on the spatial alignment device to provide for controlled movement of the insertion device to a desired position and orientation adjacent to the patient's ear to then allow for optimal, quality insertion of a cochlear implant into the patient's cochlea.

In various embodiments of the present invention, methods for placement of an object in a desired position and orientation are provided. The inventive methods include provision of a spatial alignment system comprising a spatial alignment device, a computer, software and a graphical user interface, and provision of a set of instructions to a user to manipulate input means of the spatial alignment device to cause movement of an object held by the spatial alignment device along multiple degrees of and through multiple defined increments to achieve placement of the object in a desired position and orientation.

The inventive methods may employ various embodiments of the current inventive spatial alignment system and may provide for controlled placement of medical implants or other medical devices useful in surgical applications. The inventive methods may also provide for placement of objects in other industrial applications such as manufacturing applications where controlled and defined user input is desired.

One of skill in the art will realise that while some specific embodiments are described herein with reference to cochlear implantation procedures, the inventive system will be equally applicable and beneficial in any medical or surgical application where a device or implant must be placed in a desired position and orientation for successful completion of a surgical procedure. Minimally invasive applications for placement of devices or implants come to mind. The skilled artisan will also see that the current inventive system could also be used in other industrial applications where precise placement of devices is required. For example, manufacturing applications where it is advantageous to provide a user with instructions for precise, controllable placement of an object in a desired position and orientation would benefit form the current system and approach.

One of skill in the art will also realise that, while various embodiments have been described in connection with providing instructions to a human user for controlled movement of a spatial alignment device, it is also possible to conceive of the inventive system providing instructions to a robot. For example, an autonomous robot could control the input means of the spatial alignment device according to the provided instructions. In another example, one can conceive of a variation of the inventive spatial alignment device being an end effector in a robotic system wherein the movement along multiple degrees of freedom and through defined increments was under robotic control.

These and other embodiments of the inventive system and method are described in more detail below with reference to the attached figures.

### BRIEF DESCRIPTION OF THE FIGURES

- Figure 1 shows a flow diagram for operation of a spatial alignment device according to an embodiment of the present invention.
- Figure 2 shows a conceptualized embodiment of a spatial alignment device according to the present invention in a broader configuration including a surgical arm and an adapter to a medical instrument for use in surgical applications.
- Figure 3 shows a joint as a representative input means for a spatial alignment device according to an embodiment of the present invention.
- Figure 4 shows a spatial alignment device with adjustment means for movement along multiple degrees of freedom according to an embodiment of the present invention.
- Figure 5 shows a spatial alignment device connected to a coarse positioning arm and an insertion device according to an embodiment of the present invention for placement of a medical implant in the ear of a patient.

### DETAILED DESCRIPTION OF THE INVENTION

The present invention is now described in detail in connection with its various embodiments and with reference to the attached figures and the appended claims.

In various embodiments of the present invention, a spatial alignment system for controlled placement of an object is provided. The spatial alignment system may comprise a positioning stage with a proximal end and a distal end, a spatial measurement system, a computer system comprising software and a graphical user interface. The positioning stage may be configured to (i) be selectively attachable at its proximal end to an arm in order to be placed and secured freely in space; (ii) be selectively coupled at its distal end with the object; (iii) move the coupled object in one or more degrees of freedom; and (iv) move the coupled object in defined increments of translation and rotation and by means of manual interaction. The spatial measurement system may be configured to measure the actual position and orientation in three-dimensional space of the object and/or the positioning stage relative to a target. The computer system comprises software that may be configured to (i) define a desired position and orientation for the object and/or positioning stage relative to the target; (ii) compute the relative displacement between the actual and the desired position and orientation of the positioning stage; and (iii) discretize the relative displacement into a number of increments that the positioning stage must be moved in the one or more degrees of freedom to move from its actual position and orientation to its desired position and orientation. The graphical user interface may have displayed upon it a set of instructions in a manner intelligible for a user to receive sufficient information for moving the one or more degrees of freedom of the positioning stage in discretized steps from its actual position and orientation to its desired position and orientation. The user may, accordingly, in a finite amount of time and cycles of interaction and in discretized steps, move the positioning stage to the desired position, thereby minimizing the displacement between the actual and the desired position and orientation of the positioning stage.

In various embodiments, the spatial measurement system may be an optical or an electro-magnetic measurement system.

In various embodiments, the target for the object may be a human subject, wherein the desired position and orientation in three-dimensional space of the positioning stage is a desired position and orientation with respect to the human subject.

In various embodiments, the positioning stage of the spatial alignment system may have one or more degrees of freedom and may optionally have at least 5 degrees of freedom. One of skill in the art will understand that various combinations of translational and rotational degrees of freedom are possible. Optionally, the at least 5 degrees of freedom may include at least three translational degrees of freedom and at least two rotational degrees of freedom.

In various embodiments, the spatial alignment system may further comprise an adapter configured to selectively couple the positioning stage to the object.

While those of skill in the art will understand that almost any object requiring precise positioning and orientation can be moved and placed with the current spatial alignment system, in various embodiments, the object to be placed may be a passive surgical instrument, an implantable medical device or an active surgical instrument to provide additional degrees of freedom with respect to the positioning of a surgical instrument or an implantable medical device. The skilled artisan will further understand that combinations of these options are possible. For example, the positioning stage of the present invention may be used in combination with an active surgical instrument that provides additional degrees of freedom for placement of an implantable medical device.

Solely by way of example, in one embodiment of the present invention, the positioning stage may be connected, by means of an adapter, to an active surgical instrument that operates to advance a forceps holding an implantable medical device. In certain embodiments, the implantable medical device may be a cochlear implant.

In various alternative embodiments, methods of controlled placement of an object relative to a target that may optionally be a human subject are provided. The methods include the steps of providing the inventive spatial alignment system and instructing the software to carry out various steps, including (i) defining a desired position and orientation for the positioning stage relative to a base coordinate system; (ii) computing the relative displacement between the actual and the desired position and orientation of the positioning stage; and (iii) discretizing the relative displacement into a number of increments that the positioning stage must be moved in the one or more degrees of freedom to move from its actual position and orientation to its desired position and orientation. The inventive methods may also including the steps of instructing the software to display on the graphical user interface a set of instructions in a manner intelligible for a user to receive sufficient information for moving the one or more degrees of freedom of the positioning stage in discretized steps from its actual position and orientation to its desired position and orientation and enabling the user, in a finite amount of time and cycles of interaction and in discretized steps, to move the positioning stage to the desired position, thereby minimizing the displacement between the actual and the desired position and orientation of the positioning stage.

In various method embodiments of the present invention, the user may out the set of instructions for moving the one or more degrees of freedom of the positioning stage in discretized steps from its actual position and orientation to its desired position and orientation. The inventive methods may further comprise the step of conducting a check of whether, after following the provided set of instructions, the actual position and orientation of the positioning stage is acceptably close to the desired position and orientation of the positioning stage. This check may be conducted by the user or by the computer system.

In various embodiments, the set of instructions for moving the positioning stage (and any attached object) through the various degrees of freedom to the desired position and orientation may include an independent display of the number of increments in each of the one or more degrees of freedom required to move the positioning stage from its actual position and orientation to its desired position and orientation.

Optionally, the set of instructions on the graphical user interface may also comprise a virtual representation of the positioning stage demonstrating to a user to turn selected input means on the positioning stage in which direction and by how many increments. The virtual representation may also dynamically change as the user executes the set of instructions.

In various embodiments, the software may provide the set of instructions on the graphical user interface as a sequential set of instructions for movements in the various degrees of freedom, one following the other, in a manner calculated to be most advantageous for optimal placement of the object with respect to the target.

In various embodiments, the software provides to the user a continuous feedback loop, updating for the user the progress that is being made toward the goal of placing the object in the desired position and orientation. The system provides the continuous feedback loop due to the fact that it measures, through the spatial measurement system, the actual pose of the positioning stage (and/or the object) and provides discretization and visualization at a higher temporal resolution compared to the actions of the user, thus providing instant feedback to the user.

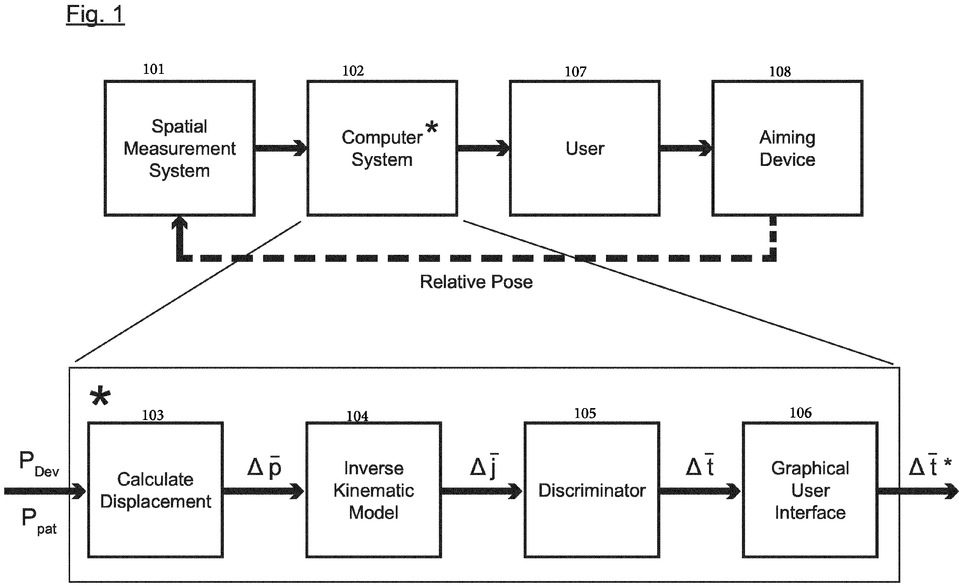

With specific reference now to the attached drawings, methods and systems related to the inventive spatial alignment device are described in detail. Figure 1 provides a flow chart for the use of a spatial alignment system for controlled placement of an object with respect to a target. Accordingly, a spatial measurement system measures 101 the actual position and orientation in three dimensional space of an object or positioning stage relative to a target and provides that position to a computer system 102. Software of the computer system 102 calculates the displacement 103 of the object or positioning stage from a desired position and orientation and feeds the displacement into an inverse kinematic model 104 of a positioning stage according to an embodiment of the present invention. The software uses the inverse kinematic model 104 and a discriminator function 105 to determine the number of increments that must be moved using the positioning stage to move the positioning stage or object to the desired position and orientation. The software then presents the incremental steps as a set of instructions on the graphical user interface 106. A user 107 can then carry out the set of instructions to change the position of the positioning stage (aiming device 108) toward the desired position and orientation. A feedback loop is then possible whereby the spatial measurement system can determine an updated actual position and orientation of the object or positioning stage with respect to the target and the system can then provide, as needed, another set of instructions for completing the movement of the object or positioning stage to the desired position and orientation.

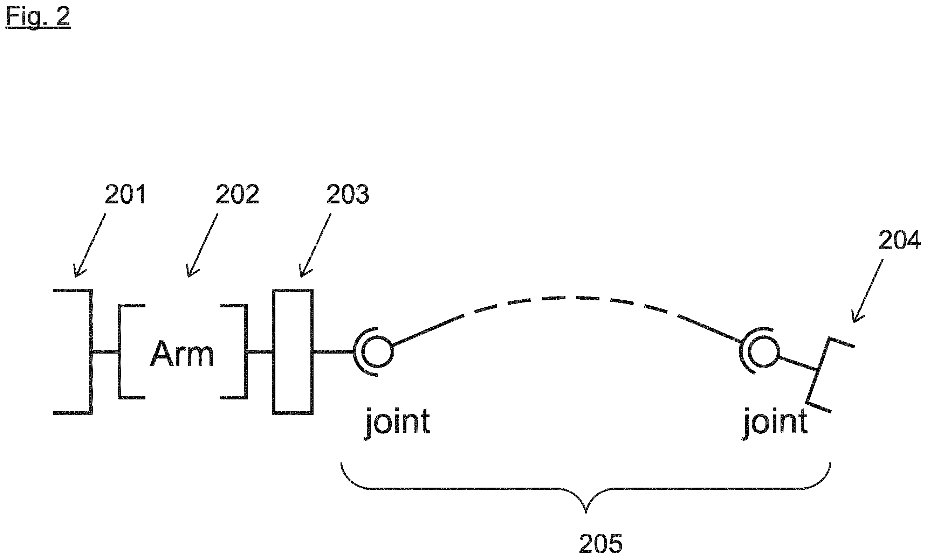

Figure 2 shows a conceptualized embodiment of a spatial alignment device according to the present invention in a broader configuration including a surgical arm and an adapter to a medical instrument for use in surgical applications. In one possible embodiment shown in Figure 2, a coarse adjustment arm 202 spans between an operating room table 201 and an adapter portion 203. The adapter portion 203 is joined to a positioning stage (or aiming device) 205 according to an embodiment of the present invention. The positioning stage has multiple joints for controlling movement along a corresponding number of degrees of freedom. The positioning stage may also be joined to an medical instrument adapter 204 for connection to a medical instrument or implantable medical device that requires placement in a precise position and orientation.

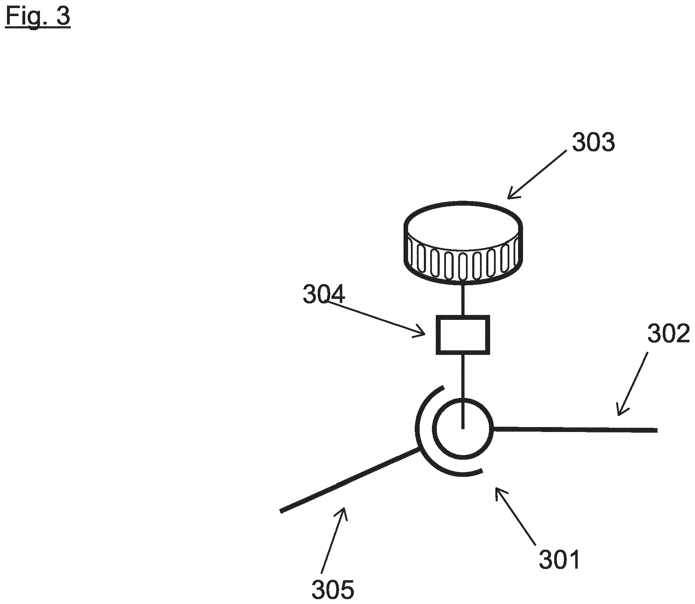

Figure 3 shows a joint as a representative input means for a spatial alignment device according to an embodiment of the present invention. Spatial alignment devices such as positioning stages or aiming devices according to embodiments of the present invention have input means provided at joints for controlling the movement of the spatial alignment device along multiple degrees of freedom. In general, the inventive spatial alignment devices have a number of joints corresponding to the number of desired degrees of freedom. In a joint such as the one shown in Figure 3, input means may take the form of a rotatable knob 303. The actual joint is shown at 301, with a corresponding input link 302 and output link 305. Rotation of the knob is mediated through a discriminator 304, that discretizes the movement of the joint 301 to defined increments or steps.

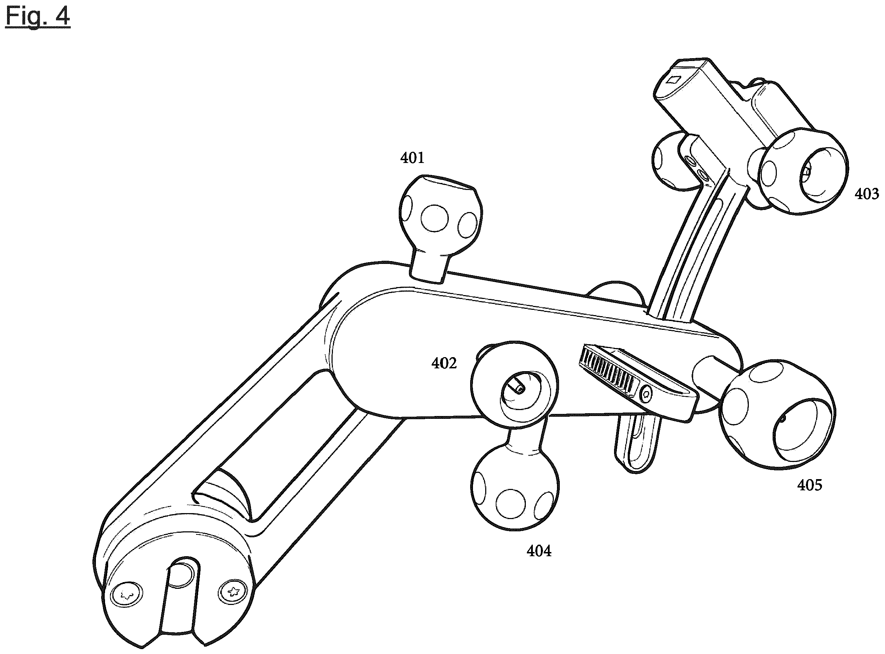

Figure 4 shows a spatial alignment device with input/adjustment means for movement along multiple degrees of freedom according to an embodiment of the present invention. The spatial alignment device shown in Figure 4 allows control of movement along five degrees of freedom through the use of knobs as input means for control of joints. Specifically, the Figure 4 spatial alignment device allows control of movement in three translational degrees of freedom and 2 rotational degrees of freedom. Tx knob 401 allows user control along the x translational axis. Ty knob 402 allows user control along the y translational axis. Tz knob 403 allows user control along the z translational axis. Rx knob 404 allows user control along the x rotational axis. Ry knob 405 allows user control along the y rotational axis.

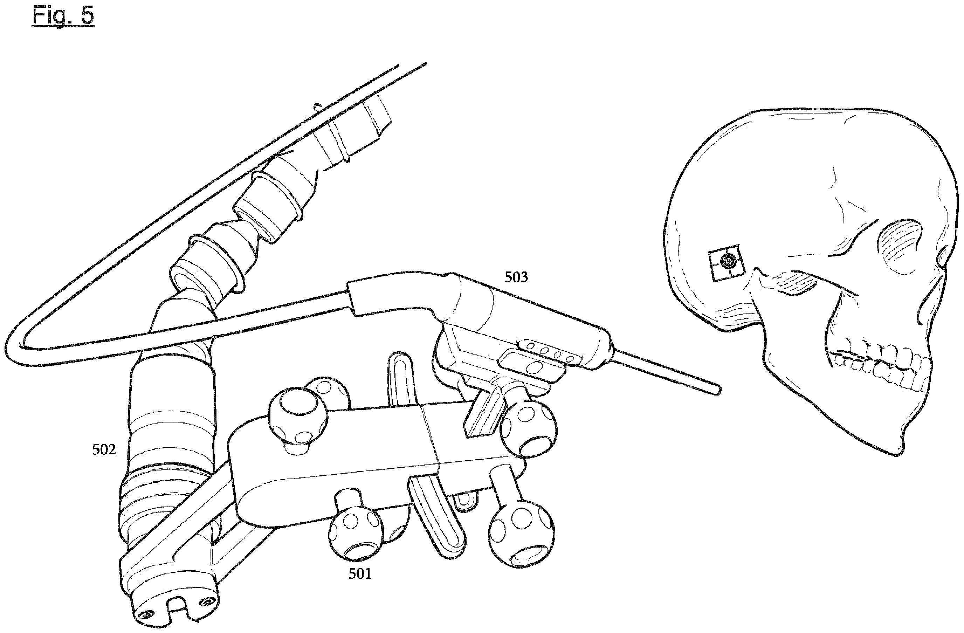

Figure 5 shows a spatial alignment device 501 connected to a coarse positioning arm 502 and an insertion device 503 according to an embodiment of the present invention for placement of a medical implant in the ear of a patient.

While this invention has been shown and described with reference to particular embodiments thereof, it will be understood by those of skill in the art that various changes in form and details may be made therein without departing from the spirit and the scope of the invention as defined by the appended claims. Solely by way of example, not by way of limitation, one of skill in the art will easily understand that the methods/systems disclosed herein can be applied to other surgical fields.

## Claims

1. A spatial alignment system for controlled placement of an object comprising:
▪ a positioning stage with a proximal end and a distal end
• configured to be selectively attachable at its proximal end to an arm in order to be placed and secured freely in space;
• configured to be selectively coupled at its distal end with the object;
• configured to move the coupled object in one or more degrees of freedom;
• configured to move the coupled object in defined increments of translation and rotation and by means of manual interaction.
▪ a spatial measurement system configured to measure the actual position and orientation in three-dimensional space of the object and/or the positioning stage relative to a target;
▪ a computer system comprising software configured to
o define a desired position and orientation for the object and/or positioning stage relative to the target;
o compute the relative displacement between the actual and the desired position and orientation of the positioning stage; and
o discretize the relative displacement into a number of increments that the positioning stage must be moved in the one or more degrees of freedom to move from its actual position and orientation to its desired position and orientation; and
▪ a graphical user interface
o upon which a set of instructions may be displayed in a manner intelligible for a user to receive sufficient information for moving the one or more degrees of freedom of the positioning stage in discretized steps from its actual position and orientation to its desired position and orientation;
▪ wherein a user may accordingly, in a finite amount of time and cycles of interaction and in discretized steps, move the positioning stage to the desired position, thereby minimizing the displacement between the actual and the desired position and orientation of the positioning stage;

2. The spatial alignment system of claim 1, wherein the spatial measurement system is a optical or an electro-magnetic measurement system.

3. The spatial alignment system of claim 1, wherein the target is a human subject.

4. The spatial alignment system of claim 3, wherein the desired position and orientation in three-dimensional space of the positioning stage is a desired position and orientation with respect to the human subject.

5. The spatial alignment system of claim 1, wherein the one or more degrees of freedom are at least 5 degrees of freedom.

6. The spatial alignment system of claim 5, wherein the at least five degrees of freedom include at least three translational degrees of freedom and at least two rotational degrees of freedom.

7. The spatial alignment system of claim 1, further comprising an adapter configured to selectively couple the positioning stage to the object.

8. The spatial alignment system of claim 7, wherein the object to be placed is selected from the group consisting of
a. a passive surgical instrument;
b. an implantable medical device;
c. an active surgical instrument to provide additional degrees of freedom with respect to the positioning of a surgical instrument or an implantable medical device; and
d. a combination thereof.

9. The spatial alignment system of claim 8, wherein the object is an active surgical instrument that advances a forceps holding an implantable medical device.

10. The spatial alignment system of claim 9, wherein the implantable medical device is a cochlear implant.
